# EUROPEAN PATENT SPECIFICATION

(11) **EP 3 999 131 B1**
(45) Date of publication and mention of the grant of the patent: **23.10.2024**
(21) Application number: 20740311.4
(22) Date of filing: 14.07.2020
(51) Int. Cl.: A61L 24/00, A61L 27/54, C08G 63/08, C08G 63/664, C08G 63/91, C08G 69/44, B33Y 80/00

(54) **MULTI-BLOCK SHAPE MEMORY BIORESORBABLE POLYMERS**
BIORESORBIERBARE MULTIBLOCK-FORMGEDÄCHTNISPOLYMERE
POLYMÈRES À BLOCS MULTIPLES, BIORÉSORBABLES, À MÉMOIRE DE FORME

(30) Priority: 19.07.2019 US 201916517192
(43) Date of publication of application: 25.05.2022
(73) Proprietor: Evonik Operations GmbH, 45128 Essen (DE)
(72) Inventor: ZHANG, Jian-Feng, Vestavia, AL 35243 (US); XUE, Teng, TIANJIN, 300071 (CN); JONES, Marshall, Scott, Bessemer, AL 35022 (US); PRABHU, Balaji, Hoover, AL 35226 (US); KARAU, Andreas, 63571 Gelnhausen (DE)
(74) Representative: Evonik Patent Association
(86) International application number: PCT/EP2020/069855
(87) International publication number: WO 2021/013629

(56) References cited:
- WO-A1-2010/091298
- WO-A1-2013/015685
- US-A- 6 160 084
- US-A1- 2003 139 567

## Description

### Field of the Invention

This invention relates to the synthesis of multi-block bioresorbable polymers bearing hard and soft polymeric segments. The invention further relates to bioresorbable polymers with shape memory properties. The invention also relates to the use of such polymers as bone filler, vascular closure and other hemostasis devices, aneurysms, mastectomy devices, and stent applications. The invention relates also to the use of such polymers for applications in fast degradation applications and 3D printing. The invention also relates to the use of such compounds as drug delivery platforms.

### Background of the Invention

Shape memory polymers (SMPs) represent a class of stimuli-responsive materials for which the response is a change in shape of an item made from the SMP based on an external stimulus such as temperature, electricity, magnetism, etc. The polymer can be deformed and subsequently fixed into a temporary shape, which would remain stable unless it is exposed to an appropriate external stimulus that triggers the polymer to recover to its original (or permanent) shape. Although various forms of external stimuli may be utilized as the recovery trigger, the most typical one is direct heating that leads to a temperature increase.

Because of the unique performance, shape memory materials are on the verge of commercialization for medical applications such as catheters, bone casts, prosthetics, self-dilating introducers, and stents. In recent years, there has been an increasing interest in shape memory thermoplastic polymers, because it is easier to adjust their transition temperature and because of their superior maximum deformation as compared to regular thermoplastic polymers. The range of temperatures for shape transition covers the human body temperature range. These attributes, together with their proven biocompatibility, competitive price, and the ease with which they can be synthesized, make SMPs exploitable for medical applications. A group of implant materials with shape memory properties has already been developed for biomedical applications.

SMPs are also good candidates for use as thin films to coat the surface of metallic biomaterials to inhibit the leakage of potentially toxic elements and improve their biocompatibility, or merely for the required sterilization of the device. This process has been shown to be beneficial in preventing the degradation of implant materials. US2003139567 discloses sequentially ordered biodegradable lactide (glycolide or lactide/glycolide)epsilon -caprolactone multi-block copolymer and process for the preparation thereof.

### Summary of the Invention

It is an object of the present invention to provide bioresorbable polymers that can be used as shape memory preferably with fast degradation for applications such as bone filler, vascular closure, stents, 3D printing, and drug delivery.

The present invention is directed to a bioresorbable polymer of Formula A 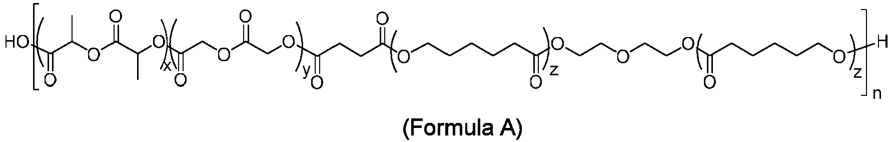 wherein:
x is between 0 and 2,000;
y is between 0 and 2,000;
x plus y is between 2 and 2000;
z is between 2 and 2,000; and
n is between 1 and 1,000.

The present invention is directed to a bioresorbable polymer of Formula B 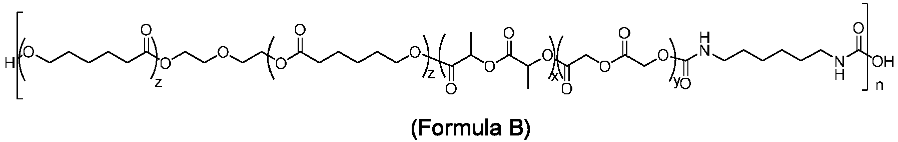 wherein:
x is between 0 and 2,000;
y is between 0 and 2,000;
x plus y is between 2 and 2000;
z is between 2 and 2,000; and
n is between 1 and 1,000.

In still another aspect, disclosed is a composition comprising: a bioresorbable polymer of Formula A, or B, or a mixture thereof.

In still another aspect, disclosed is a composition comprising: a bioresorbable polymer of Formula A, or B, or a mixture thereof; and an additive.

Additional advantages will be set forth in part in the description that follows, and in part will be obvious from the description, or can be learned by practice of the aspects described below. The advantages described below will be realized and attained by means of the elements and combinations particularly pointed out in the appended claims. It is to be understood that both the foregoing general description and the following detailed description are exemplary and explanatory only and are not restrictive.

### Brief Description of the Drawings

The above and other objects, features and advantages of the present invention will be more clearly understood from the following detailed description taken in conjunction with the accompanying drawings, in which:
FIG. 1. is a reaction scheme showing the synthesis of Formula A,
FIG. 2. is a reaction scheme showing the synthesis of Formula B,
FIG. 3. depicts shape fixation and recovery as a function of time of PLLA-PCL multiblock copolymer in a temperature range of -60°C to 40°C,
FIG. 4. depicts shape fixation and recovery as a function of temperature of PLLA-PCL multiblock copolymer in a temperature range of -60°C to 40°C.

### Detailed Description of the invention

Before the present compounds and processes are disclosed and described, it is to be understood that the aspects described herein are not limited to specific processes, compounds, synthetic methods, articles, devices, or uses as such can, of course, vary. It is also to be understood that the terminology used herein is for the purpose of describing particular aspects only and, unless specifically defined herein, is not intended to be limiting.

Disclosed herein are bioresorbable polymers bearing hard and soft polymeric segments. The disclosed bioresorbable polymers provide the advantage of a biodegradable profile, and the process ability with additives to form composites. Another advantage is that it can be extended to a vast range of applications, including shape memory, bone filler, vascular closure, stent, fast degradation applications, 3D printing, and drug delivery platforms etc.

### Definition of Terms

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present invention. All publications, patent applications, patents and other references mentioned herein are incorporated by reference in their entirety. The materials, methods, and examples disclosed herein are illustrative only and not intended to be limiting.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "an" and "the" include plural references unless the context clearly dictates otherwise. The present disclosure also contemplates other embodiments "comprising," "consisting of" and "consisting essentially of," the embodiments or elements presented herein, whether explicitly set forth or not.

The conjunctive term "or" includes any and all combinations of one or more listed elements associated by the conjunctive term. For example, the phrase "an apparatus comprising A or B" may refer to an apparatus including A where B is not present, an apparatus including B where A is not present, or an apparatus where both A and B are present. The phrases "at least one of A, B, ... and N" or "at least one of A, B, ... N, or combinations thereof' are defined in the broadest sense to mean one or more elements selected from the group comprising A, B, ... and N, that is to say, any combination of one or more of the elements A, B, ... or N including any one element alone or in combination with one or more of the other elements which may also include, in combination, additional elements not listed.

The modifier "about" used in connection with a quantity is inclusive of the stated value and has the meaning dictated by the context (for example, it includes at least the degree of error associated with the measurement of the particular quantity). The modifier "about" should also be considered as disclosing the range defined by the absolute values of the two endpoints. For example, the expression "from about 2 to about 4" also discloses the range "from 2 to 4." The term "about" may refer to plus or minus 10% of the indicated number. For example, "about 10%" may indicate a range of 9% to 11 %, and "about 1" may mean from 0.9-1.1. Other meanings of "about" may be apparent from the context, such as rounding off, so, for example "about 1" may also mean from 0.5 to 1.4.

The term "wt. %" means weight percent.

The term "w/w" means weight per weight.

For the purposes of the present invention, the term "shape memory effect" refers to polymers that have the ability to return from a deformed state (temporary shape) to their original (permanent) shape induced by an external stimulus (trigger), such as temperature change.

For the purposes of the present invention, the term "biodegradable" refers to polymers that dissolve or degrade in vivo within a period of time that is acceptable in a particular therapeutic situation. Such dissolved or degraded product may include a smaller chemical species. Degradation can result, for example, by enzymatic, chemical and/or physical processes. Biodegradation takes typically less than five years and usually less than one year after exposure to a physiological pH and temperature, such as a pH ranging from 6 to 9 and a temperature ranging from 22°C to 40°C.

For the purposes of the present invention, the term "3D printed part" refers to a part printed by a 3D printer. A 3D printer includes, but are not limited to, bioplotter, fused filament fabrication (FFF), selective laser sintering (SLS), and stereolithography (SLA). A 3D printed part can also be a bioprinted part.

For the purposes of the present invention, the term "thermal printer" refers to a printer that can print thermoplastics. A thermal printer includes, but are not limited to, fused filament fabrication (FFF), and binder jetting.

The term "biological tissues" include, but are not limited to, human soft tissues, skin, subcutaneous layer, mucous membranes, cartilage, ligaments, tendons, muscle tissues, blood vessels, human organs, cardiac muscle tissues, heart valves, nervous tissues, pericardium, pleurae, and peritoneum.

Suitable biodegradable polymers of the invention, which can be further present in the composition in addition to bioresorbable polymers A and/or B, include without limitation poly(lactide), a poly(glycolide), a poly(lactide-co-glycolide), a poly(caprolactone), a poly(orthoester), a poly(phosphazene), a poly(hydroxybutyrate) a copolymer containing a poly(hydroxybutarate), a poly(lactide-co-caprolactone), a polycarbonate, a polyesteramide, a polyanhydride, a poly(dioxanone), a poly(alkylene alkylate), a copolymer of polyethylene glycol and a polyorthoester, a biodegradable polyurethane, a poly(amino acid), a polyamide, a polyesteramide, a polyetherester, a polyacetal, a polycyanoacrylate, a poly(oxyethylene)/poly(oxypropylene) copolymer, polyacetals, polyketals, polyphosphoesters, polyhydroxyvalerates or a copolymer containing a polyhydroxyvalerate, polyalkylene oxalates, polyalkylene succinates, poly(maleic acid), and copolymers, terpolymers, combinations thereof.

The invention refers to bioresorbable polymers according to Formula A and Formula B as set out in claim 1 and 2.

The x value of the bioresorbable polymer A or B is between 0 and 2,000. Preferably, the x value of the bioresorbable polymer A or B is between 1 and 800. More preferably, the x value is between 2 and 500.

The y value of the bioresorbable polymer A or B is between 0 and 2,000. Preferably, the n value of the bioresorbable polymer A or B is between 1 and 800. More preferably, the x value is between 2 and 500.

The x plus y value of the bioresorbable polymer A or B is between 2 and 2,000. Preferably, the x plus y value is between 20 and 800. More preferably, the x plus y value is between 20 and 500.

The z value of the bioresorbable polymer A or B is between 2 and 2,000. Preferably, the n value of the bioresorbable polymer A or B is between 10 and 800. More preferably, the z value of the bioresorbable polymer A or B is between 15 and 300.

The n value of the bioresorbable polymer A or B is between 1 and 1,000. Preferably, the n value of the bioresorbable polymer A or B is between 2 and 500. More preferably, the n value is between 5 and 300.

The ratio between x plus y value and z value is between 1 and 100. Preferably, the ratio between x plus y value and z value is between 2 and 50. More preferably, the ratio between x plus y value and z value is between 3 and 30.

The biodegradable or bioresorbable polymers can comprise one or more residues of lactic acid, glycolic acid, lactide, glycolide, caprolactone, hydroxybutyrate, hydroxyvalerates, dioxanones, polyethylene glycol (PEG), polyethylene oxide, or a combination thereof. In some aspects, the biodegradable or bioresorbable polymers comprise one or more lactide residues. The polymer can comprise any lactide residue, including all racemic and stereospecific forms of lactide, including, but not limited to, L-lactide, D-lactide, and D,L-lactide, or a mixture thereof. Useful polymers comprising lactide include, but are not limited to poly(L-lactide), poly(D-lactide), and poly(DL-lactide); and poly(lactide-co-glycolide), including poly(L-lactide-co-glycolide), poly(D-lactide-co-glycolide), and poly(DL-lactide-co-glycolide); or copolymers, terpolymers, combinations, or blends thereof. Lactide/glycolide polymers can be conveniently made by melt polymerization through ring opening of lactide and glycolide monomers. Additionally, racemic DL-lactide, L-lactide, and D-lactide polymers are commercially available. The L-polymers are more crystalline and resorb slower than DL- polymers. In addition to copolymers comprising glycolide and DL-lactide or L-lactide, copolymers of L-lactide and DL-lactide are commercially available. Homopolymers of lactide or glycolide are also commercially available.

When poly(lactide-co-glycolide), poly(lactide), or poly(glycolide) is used, the amount of lactide and glycolide in the polymer can vary. For example, the biodegradable or bioresorbable polymers can contain 0 to 100 mole %, 40 to 100 mole %, 50 to 100 mole %, 60 to 100 mole %, 70 to 100 mole %, or 80 to 100 mole % lactide and from 0 to 100 mole %, 0 to 60 mole %, 10 to 40 mole %, 20 to 40 mole %, or 30 to 40 mole % glycolide, wherein the amount of lactide and glycolide is 100 mole %. In a further aspect, the biodegradable polymer can be poly(lactide), 95:5 poly(lactide-co-glycolide) 85:15 poly(lactide-co-glycolide), 75:25 poly(lactide-co-glycolide), 65:35 poly(lactide-co-glycolide).

In the preparation of the molecular backbone, the polymerization steps can be carried out using a catalytically-effective amount of a catalyst. The formation of the polymer of cyclic esters can be carried out with any suitable catalyst known to polymerize cyclic esters. The polymerization catalyst can be metallic or non-metallic, including a variety of non-metallic organic catalysts. Suitable metal catalysts include zinc powder, tin powder, aluminum, magnesium and germanium, metal oxides such as tin oxide (II), antimony oxide (III), zinc oxide, aluminum oxide, magnesium oxide, titanium oxide (IV) and germanium oxide (IV), metal halides such as tin chloride (II), tin chloride (IV), tin bromide (II), tin bromide (IV), antimony fluoride (III), antimony fluoride (V), zinc oxide, magnesium chloride and aluminum chloride, sulfates such as tin sulfate (II), zinc sulfate and aluminum sulfate, carbonates such as magnesium carbonate and zinc carbonate, borates such as zinc borates, organic carboxylates such as tin acetate (II), tin octanoate (II), tin lactate (II), zinc acetate and aluminum acetate, organic sulfonates such as tin trifluoromethane sulfonate (II), zinc trifluoromethane sulfonate, magnesium trifluoromethane sulfonate, tin (II) methane sulfonate and tin (II) p-toluene sulfonate. Dibutyltin dilaurate (DBTL), Sb₂O₃, Ti(IV) butoxide Ti(IV) isopropoxide, and others. The polymerization catalyst can also be a non-metallic acids, such as an organic acid. The organic acid can be a weak acid or a strong acid. Examples of suitable organic acids include acetic acid, methane sulfonic acid, ethane sulfonic acid, 1-propane sulfonic acid, 1-butane sulfonic acid, trifluoromethane sulfonic acid, benzene sulfonic acid, p-toluene sulfonic acid, p-xylene-2-sulfonic acid, naphthalene-1-sulfonic acid and naphthalene 2-sulfonic acid, and stronger acids such as hydrochloric acid, sulfuric acid, glacial acetic acid, and phosphoric acid. In a preferred aspect of the process, the polymerization catalyst is tin octanoate (II). In the preparation of the molecular backbone, the polymerization steps can be carried out also using a chain initiator. Various initiator agents that can be used for the coupling reaction include, but are not limited to, glycolic acid, polyethylene glycol (PEG), and polyols.

The addition of the catalyst in the reaction mixture can be accomplished by adding the catalyst neat or dissolved in a solvent. Suitable solvents that can be used for dissolving the catalyst include, but not limited to, dimethyl sulfoxide, dimethyl formamide, and toluene.

The process comprises polymerizing a cyclic ester by heating a molten reaction mixture comprising the cyclic ester at a temperature of from about 100 °C to about 300 °C for a time ranging from about 0.5 hours to about 24 hours to form the polymeric ester in the molten reaction mixture.

According to this aspect of the process, the cyclic esters are heated at a temperature of from about 80 °C to about 250 °C., preferably from about 100 °C to about 200 °C, under reduced pressure, atmospheric pressure or sufficient pressure in the presence of an optional polymerization catalyst and initiator to conduct a polymerization reaction. After the formation of the polymers of the cyclic esters, the polymers can be undergone to a further carboxylation reaction step to form a carboxylated polymer. The carboxylation reaction can be carried out in the same or different reaction vessel. The two-step process can be carried out in a single reaction vessel (one-pot). The preparation of the carboxylate polymers can typically be accomplished by using organic cyclic anhydrides as reactant. Various organic cyclic anhydrides that can be used for the carboxylation of the polymeric backbone include, but are not limited to, succinic anhydride, glutaric anhydride, adipic anhydride, pimelic anhydride and maleic anhydride. Accordingly, the process comprises adding to the molten reaction mixture an appropriate amount of the cyclic anhydride and let the reaction mixture stir for a time ranging from about 0.5 hours to about 72 hours at a temperature of from about 80 °C to about 250 °C, preferably from about 100 °C to about 200 °C to form the carboxylated polymer. The residual monomers and cyclic anhydride are distilled away from the reaction at a pressure of about 1 torr to 10 torr, preferably from 1 torr to 5 torr. The carboxylated polymer is removed from the reaction vessel and purified by precipitation. For example, the carboxylated polymer is dissolved in a solvent and then precipitated by adding a non-solvent for the carboxylated polymer. Various organic solvents that can be used as solvent for the carboxylated polymer include, but not limited to, acetone, chloroform, dichloromethane, dimethylsulphoxide, dimethyl formamide. Various solvents that can be used as a non-solvent for the carboxylated polymer include, but not limited to, ethanol, methanol, water, cyclohexane, hexane, and pentane.

The preparation of the carboxylate forms of the polymer backbones can typically be accomplished by using organic cyclic anhydrides as reactant. Various organic cyclic anhydrides that can be used for the carboxylation of the polymeric backbone include, but are not limited to, succinic anhydride, glutaric anhydride, adipic anhydride, pimelic anhydride and maleic anhydride. Accordingly, the process comprises stirring a liquid reaction medium comprising the molecular backbone at a temperature of from about 20 °C to about 50 °C for a time ranging from about 0.5 hours to about 72 hours to form the carboxylated polymer backbones in the liquid reaction medium.

The carboxylated polymer backbones is removed from the liquid reaction medium by precipitation, for example by adding a non-solvent for the carboxylated polymer backbones to precipitate the carboxylated polymer backbones out from the mixture or by recrystallizing the carboxylated polymer backbones. The carboxylated polymer backbones can also be recrystallized using a solvent such as ethyl acetate, dichloromethane, chloroform, diethyl ether or a mixture thereof. The carboxylated polymer backbones can also be further purified by separating it from a mixture by centrifugal precipitation or decantation. The carboxylated polymer backbones can also be washed with a non-solvent for the carboxylated polymer backbones such as cyclohexane, methanol, ethanol or ether.

The coupling of the different polymer backbones can typically be accomplished by using an activating agent to mediate the coupling reaction. Various activating agents that can be used for the coupling reaction include, but are not limited to, 1-ethyl-3-(3-dimethylaminopropyl)carbodiimide (EDC), dicyclohexylcarbodiimide (DCC), N,N-diisopropyl-carbodiimide (DIP), benzotriazollyl-oxy-tris-( dimethyl amino )phosphonium hexa-fluorophosphate (BOP), N-dimethyl-amino pyridine (DMAP) hydroxybenzotriazole (HOBt), and N-methylmorpholine (NMM), including a mixture thereof. The coupling reaction can be carried out in N-methylpyrrolidone (NMP), DMF or in dichloromethane (DCM). Accordingly, the process comprises stirring a liquid reaction medium comprising the carboxylated polymer at a temperature of from about 20 °C to about 50 °C for a time ranging from about 0.5 hours to about 72 hours to form the multiblock-copolymer in the liquid reaction medium.

The multiblock-copolymer is removed from the liquid reaction medium by precipitation, for example by adding a non-solvent for the multi block-copolymer to precipitate the multiblock-copolymer out from the mixture or by recrystallizing the multiblock-copolymer. The multiblock-copolymer can also be recrystallized using a solvent such as ethyl acetate, dichloromethane, chloroform, diethyl ether or a mixture thereof. The multiblock-copolymer can also be further purified by separating it from a mixture by centrifugal precipitation or decantation. The multiblock-copolymer can also be washed with a non-solvent for the multiblock-copolymer such as cyclohexane, methanol, ethanol or ether.

The preparation of the multiblock-copolymer can also be accomplished by using the desired isocyanate functional moieties as reactant. The coupling of the multiblock-copolymer with the isocyanate functional moiety can typically be carried out using a catalytically-effective amount of a catalyst. The formation of the urethane group to link the multi block-copolymer to the functional moiety can be carried out with any suitable catalyst known to promote isocyanate reactivity. The reaction catalyst can be metallic or non-metallic, including a variety of non-metallic organic catalysts. Suitable metal catalysts include, but not limited to, organo tin compounds such as tin acetate (II), tin octanoate (II), tin lactate (II), tin (II) methane sulfonate and tin (II) p-toluene sulfonate, dibutyltin dilaurate (DBTL). The reaction catalyst can also be a non-metallic compound, such as an organic base. The organic base can be a weak base or a strong base. Examples of suitable organic base include, but are not limited to triethyl amine (TEA), DABCO (1,4-diazabicyclo[2.2.2]octane), dimethylcyclohexylamine (DMCHA), dimethylethanolamine (DMEA), 1,8-diazabicyclo[5.4.0]undec-7-ene (DBU) and 4-dimethylaminopyridine (DMAP).

Accordingly, the process comprises stirring a liquid reaction medium comprising the multiblock-copolymer at a temperature of from about 20 °C to about 100 °C for a time ranging from about 0.5 hours to about 72 hours to form the multiblock-copolymer in the liquid reaction medium.

The multiblock-copolymer is removed from the liquid reaction medium by precipitation, for example by adding a non-solvent for the functionalized multiblock-copolymer to precipitate the functionalized multiblock-copolymer out from the mixture or by recrystallizing the functionalized multiblock-copolymer. The multiblock-copolymer can also be recrystallized using a solvent such as acetone, ethyl acetate, dichloromethane, chloroform, diethyl ether or a mixture thereof. The multiblock-copolymer can also be further purified by separating it from a mixture by centrifugal precipitation or decantation. The functionalized multiblock-copolymer can also be washed with a non-solvent for the multiblock-copolymer such as cyclohexane, methanol, ethanol or ether.

The solvent used in the present invention include, but are not limited to acetone, chloroform, dichloromethane, dimethylsulfoxide, dimethyl formamide, polyethylene glycol, or N-Methyl-2-Pyrrolidone (NMP).

The non-solvent used in the present invention include, but are not limited to ethanol, methanol, water, cyclohexane, hexane, pentane, hydrogen peroxide, diethyl ether, tert-butyl methyl ether (TBME), phosphate buffer saline solution (PBS), or a mixture thereof.

Suitable additives include, but are not limited to growth factors, vitamins, peptides, biologics, amino acids, antibiotics, and antiviral agents.

In another embodiment, additives include, but are not limited to alendronate, olpadronate, etidronate, colecalciferol (vitamin D), tocopherol (vitamin E), pyridoxin (vitamin B6), cobalamin (vitamin B12), platelet-derived growth factor (PDGF), glycine, lysine, penicillin, cephalosporin, tetracycline, lamivudine, and zidovudine.

Suitable isocyanates used in the present invention include, but are not limited to aliphatic isocyanate, cycloaliphatic isocyanates, L-lysine diisocyanate, and hexamethylene diisocyanate.

The multifunctional molecules can, for example, include at least one of polyethylene glycol, a polyamino acid (typically, greater than 50 linked amino acids and including, for example, proteins and/or polypeptides), an aliphatic polyester (including, for example, polylactic acid, polyglycolic acid and/or polycaprolactone), a saccharide (including, for example, a sugar), a polysaccharide (for example, starch), an aliphatic polycarbonate, a poly amine (including, for example, Polyethylenimine), a polyanhydride, a steroid (for example, hydrocortisone), glycerol, ascorbic acid, an amino acid (for example, lysine, tyrosine, serine, and/or tryptophan), or a peptide (typically, 2 to 50 linked amino acids), an inorganic particle (for example bioglass, hydroxyapatite, ceramic particles).

In another embodiment, thermal processing incudes, but are not limited to, single screw extrusion, twin screw extrusion, compression molding, thermal forming, additive manufacturing or 3D printing, and injection molding.

The typically degradation profiles of Formula A, Formula B, or a mixture thereof can be at least two weeks, at least one month, at least 3 months, at least 6 months, at least 9 months, at least 12 months, at least 18 months, or at least 24 months.

In a 3D printing application using a bioplotter, the method of producing a 3D printed part with the polymer backbone, Formula A, Formula B, or a mixture thereof includes making a polymer solution with the said polymers or polymer pellets to be printed. The polymer solution is prepared and stored in a cartridge compatible with the 3D printer. The additive solution is prepared and stored in appropriate conditions. The cartridge can be filled with polymer pellets for printing. A solid model is developed with the desired print geometry. The solid model is prepared for printing by performing a 'slicing' operation. The slicing operation separates the solid part geometry into the multiple layers that the printer is going to print. The layer height of the slices is determined by the operator and tip opening diameter. A petri dish mount is secured to the platform. The petri dish used as a printing surface is placed within the mount. The prepared print geometry file is imported into the 3D printer software. The print is prepared by assigning a material to be used for the print and assigning a pattern to be used for the print infill. Additional factors are altered in this stage for the printing operation, but the two most basic changes are assigning a material to print with and a pattern for the print infill. A tip of desired diameter is added to the polymer solution cartridge and the cartridge is placed into the print head of the 3D printer. The printing surface of the petri dish is prepared by spraying a uniform layer of the additive. The print head containing the polymer solution is calibrated, and initial printing parameters are estimated and placed into the material profile in the 3D printer software. The printing operation is started by the operator. The printing head of the 3D printer moves in the x and y direction to print the part geometry. Inbetween layers, the polymer is allowed to cure for a minimum of 30 seconds. The print head then raises (z) and prints the next layer of the geometry. This process is repeated until the entire part has been printed.

In a 3D printing application using FFF, the method produces a 3D printed part with polymer material of Formula A, Formula B, biodegradable polymers, or a mixture thereof. A solid model is developed with the desired print geometry. The solid model is prepared for printing by performing a 'slicing' operation. The slicing operation separates the solid part geometry into the multiple layers that the printer is going to print. The layer height of the slices is determined by the operator and tip opening diameter. The prepared print geometry file is imported into the 3D printer software. The print is prepared by assigning the polymer material to be used for the print and assigning a pattern to be used for the print infill. Polymer material feeds into the temperature-controlled FFF extrusion head, where it is heated to a semi-liquid state. The head extrudes and deposits the material in ultra-thin layers onto a fixtureless base. The head directs the material into place with precision. The material solidifies, laminating to the preceding layer. Parts are fabricated in layers, where each layer is built by extruding a small bead of material, or rod, in a particular laydown pattern, such that the layer is covered with the adjacent roads. After a layer is completed, the height of the extrusion head is increased and the subsequent layers are built to construct the part.

In a 3D printing application using binder jetting, the method produces a 3D printed part with polymer material powder of Formula A, Formula B, biodegradable polymers, or a mixture thereof. A solid model is developed with the desired print geometry. The solid model is prepared for printing by performing a 'slicing' operation. The slicing operation separates the solid part geometry into the multiple layers that the printer is going to print. The layer height of the slices is determined by the operator and tip opening diameter. The prepared print geometry file is imported into the 3D printer software. The print is prepared by assigning the polymer material to be used for the print and assigning a pattern to be used for the print infill. The polymer materials powder is provided, then an amount of a binder is deposited onto the powder to produce an unfinished layer. The process is repeated to produce a three-dimensional unfinished model. The unfinished model is then sintered to produce a three-dimensional 3D printed part having a functionally-graded structure

### Examples

The following examples are put forth so as to provide those of ordinary skill in the art with a complete disclosure and description of how the compounds, compositions, articles, devices and/or methods claimed herein are made and evaluated, and are intended to be purely exemplary of the invention and are not intended to limit the scope of what the inventors regard as their invention. Efforts have been made to ensure accuracy with respect to numbers (e.g., amounts, temperature, etc.), but some errors and deviations should be accounted for. Unless indicated otherwise, parts are parts by weight, temperature is in °C. or is at ambient temperature, and pressure is at or near atmospheric.

Materials of Formula A and B may be prepared according to the following reaction schemes. In general, the material of this invention can be made by processes which include processes analogous to those known in the chemical arts, particularly in light of the description contained therein. Certain processes for the manufacture of the compounds of this invention are provided as further features of the invention and are illustrated by the following reaction schemes.

### PREPARATION OF EXAMPLE 1

### Synthesis of Formula A (PLLA-PCL multi-block copolymer)

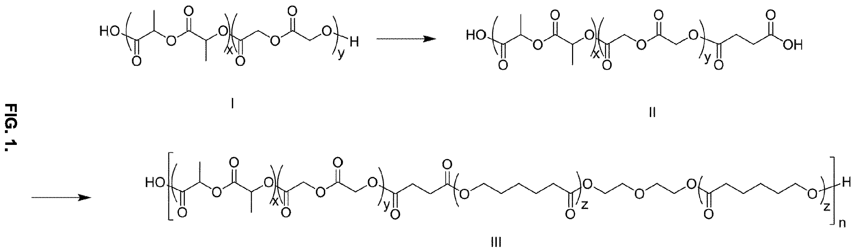

Scheme 1 depicted in FIG. 1. refers to the preparation of Formula A. 10 g PLA (substrate I), succinic anhydride (0.5 g, 5 mmol), DMAP (0.6g, 5 mmol), and triethylamine (0.7 mL, 5 mmol) were dissolved in 100ml 1,4-dioxane and left overnight at room temperature. The filtered solution was precipitated by cold diethyl ether, re-dissolved in DCM and precipitated, washed by cold diethyl ether and dried for 3 days under vacuum to yield dicarboxylic acid-terminated PLA (product II).

2 g dicarboxylic acid-terminated PLA (substrate II), PCL-diol 0.4 g, DCC (0.12 g, 0.6 mmol) and DMAP (0.07 g, 0.6 mmol) were dissolved in DCM (40 mL) and react at room temperature for 3 days. The precipitated dicyclohexylurea is filtered off, the solution is poured into cold diethyl ether and the precipitate washed with excess of diethyl ether. Drying under vacuum at room temperature for 2 days to yield Formula A (product III).

### PREPARATION OF EXAMPLE 2

### Synthesis of Formula B

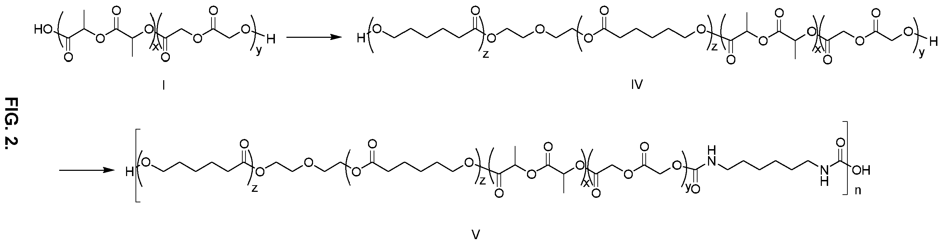

Scheme 2 depicted in FIG. 2. refers to the preparation of Formula B.

10 g PLA (substrate I), 2 g PCL-diol, DCC (0.3 g, 1.5 mmol) and DMAP (0.17 g, 1.5 mmol) were dissolved in DCM (100 mL) and react at room temperature for 3 days. The precipitated dicyclohexylurea is filtered off, the solution is poured into cold diethyl ether and the precipitate washed with excess of diethyl ether. Drying under vacuum at room temperature for 2 days to yield diblock PLA-PCL (product IV).

5 g diblock PLA-PCL (substrate IV), were dissolved in 50 mL dry dioxane and HDI (77 mg, 0.46 mmol) was added to the dissolved triblock, followed by stannous octanoate catalyst (0.075 g, 0.18 mmol). The reaction was conducted at 82°C for 3 h, under a dry nitrogen atmosphere and mechanical stirring (100 rpm). The resulting polymer was precipitated into ether and washed with excess of diethyl ether. Drying under vacuum at room temperature for 2 days to yield Formula B (product V).

### PREPARATION OF EXAMPLE 3

### Processing of Formula A or B

Polymer consisting of Formula A or Formula B was thermally processed by a HAAKE MinJet Pro piston injection molding system to dog-bone shape following ISO 527-1 BB. Polymer Formula A was vacuum dried overnight before thermal processing. The melting and mold temperatures were 95°C and 25°C, respectively. After melting, the material was injected into the mold with an injection pressure of 65 MPa for 8 seconds and a hold pressure of 40 MPa for 4 seconds.

Polymer Formula A use the same thermal processing procedure and parameters to get dog-bone shape specimens following ISO 527-1BB.

### Testing

Shape memory property of the materials consisting Formula A or Formula B were evaluated by a Dynamic Mechanical Analyzer (DMA, Q-800, TA Instruments). Narrow section of injection molded specimens was cut to straight rectangular shape (18 × 2 × 1.5 mm) and mounted to the DMA with a pair of tensile clamps. The shape memory testing was performed by a controlled force method. The specimens were then heated to 40°C, held at 40°C for 10 min, then applied 0.3 - 15 N force on the specimen. Afterwards, the specimens were cooled to -60°C with applied constant force, held at -60°C for 30 min, the force was unloaded and the specimen was heated to 40°C and held at 40°C for 30 min. Shape recovery rate is determined by Rr(%)=(εᵤ-εₚ)/( εₘ-εₚ)×100, where εᵤ, εₚ and εₘ represent the fixed strain after unloading, the permanent strain after heat-induced recovery, and the temporal strain achieved by deformation. All these strains were measured and recorded by the DMA.

### Results

**Table 1. Shape memory recovery rate of the bioresorbable polymers tested by DMA.**

| **Composition** | **Shape recovery rate** |
|---|---|
| Formula A | 90% |
| Formula B | 50% |
| PLLA (Resomer L 210S) | 25% |
| PCL (Resomer C 209) | 27% |
| Blend PLLA (Resomer L 210S) & PCL (Resomer C 209) 5:1 | 0% |
| PLLA-co-PCL (Resomer LC 703S) | 28% |

The multi-block copolymer has higher shape recovery rate than the homocomponent polymer, random copolymer and physical mixture of polymers.

**Table 2. Tensile properties of the bioresorbable multiblock polymers PLLA-PCL-PLLA, where the PLLA segment length is 10k, and PCL segment chain length is 2k.**

| Polymer | Tensile strength (MPa) | Elastic Modulus (MPa) | Elongation at break (%) | Elongation at maximum load (%) |
|---|---|---|---|---|
| PLLA-PCL-PLLA | 5.57 | 64.6 | 145.2 | 18.8 |

The multi-block copolymer of PLLA-PCL-PLLA is flexible.

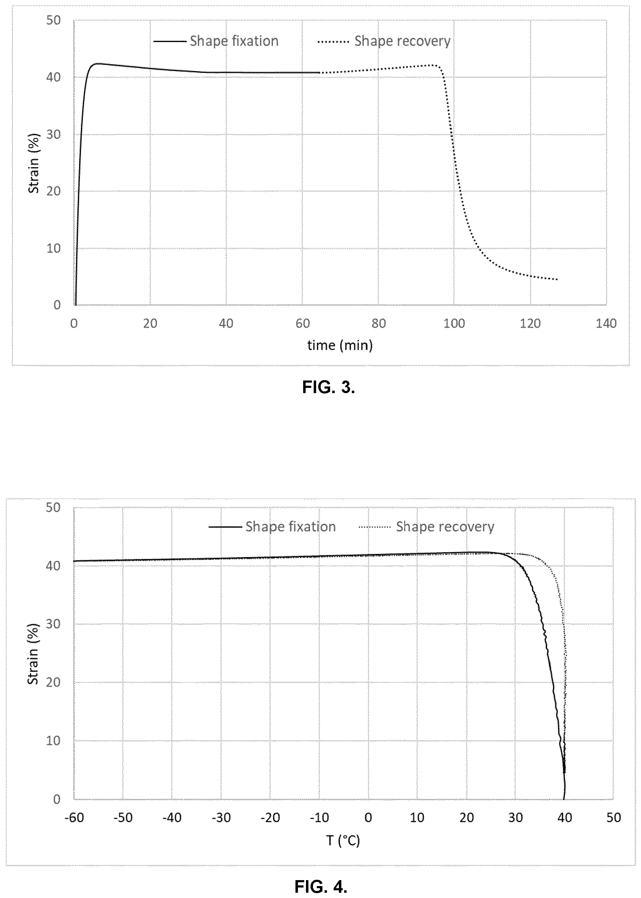

FIG. 3. depicts shape fixation and recovery as a function of time of PLLA-PCL multiblock copolymer in a temperature range of -60°C to 40°C. The PLLA segment length is 10k, and PCL segment chain length is 2k. The polymer exhibits rapid shape recovery from about 42% strain to 8% strain.

FIG. 4. depicts shape fixation and recovery as a function of temperature of PLLA-PCL multiblock copolymer in a temperature range of -60°C to 40°C. The PLLA segment length is 10k, and PCL segment chain length is 2k. The polymer exhibits narrow recovery temperature around 40°C.

Item 1 is a bioresorbable polymer of Formula A 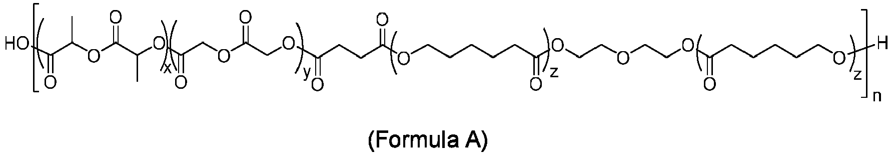 wherein:
x is between 0 and 2,000;
y is between 0 and 2,000;
x plus y is between 2 and 2000;
z is between 2 and 2,000; and
n is between 1 and 1,000.

Item 2 is a bioresorbable polymer of Formula B 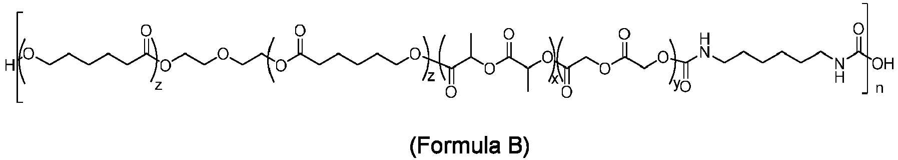 wherein:
x is between 0 and 2,000;
y is between 0 and 2,000;
x plus y is between 2 and 2000;
z is between 2 and 2,000; and
n is between 1 and 1,000.

Item 3 is a composition comprising:
a bioresorbable polymer of Formula A of item 1, Formula B of item 2, or a mixture thereof.

Item 4 is the composition of item 3, wherein the composition further comprises an additive, wherein the additive is an antimicrobial agent, an antibacterial agent, a growth factor, a vitamin, a biologic, an antibiotic, an antiviral agent, antifungal agent, alendronate, olpadronate, etidronate, colecalciferol (vitamin D), tocopherol (vitamin E), pyridoxin (vitamin B6), cobalamin (vitamin B12) platelet-derived growth factor (PDGF), glycine, lysine, penicillin, cephalosporin, lamivudine, tetracycline, and zidovudine, polyethylene glycol, a polyamino acid (typically, greater than 50 linked amino acids and including, for example, proteins and/or polypeptides), an aliphatic polyester (including, for example, polylactic acid, polyglycolic acid and/or polycaprolactone), a saccharide (including, for example, a sugar), a polysaccharide (for example, starch), an aliphatic polycarbonate, a poly amine (including, for example, Polyethylenimine), a polyanhydride, a steroid (for example, hydrocortisone), glycerol, ascorbic acid, an amino acid (for example, lysine, tyrosine, serine, and/or tryptophan), or a peptide (typically, 2 to 50 linked amino acids), an inorganic particle (for example, bioglass, hydroxyapatite, ceramic particles), poly-I-lysine (PLL), poly-d-lysine (PDL), poly-d,I-lysine (PDLL), poly-I-cysteine, poly-d-cysteine, poly-d,I-cysteine, short oligomers of I-lysine, d-lysine, I- cysteine, d-cysteine, an amino functionalized PEG, an amino functionalized inorganic particle (bioglass, hydroxyapatite, tetracalcium phosphate), a tin catalyst, or a mixture thereof.

Item 5 is a process for preparing a bioresorbable polymer of Formula A of item 1, and/or a bioresorbable polymer of Formula B of item 2 comprising the steps of mixing a polymer backbone with a functional group precursor to form a mixture; and adding a linker to the mixture to form the bioresorbable polymer.

Item 6 is a bioresorbable polymer composition with shape memory properties comprising the bioresorbable polymer of the present invention or of item 3.

Item 7 is a bioresorbable polymer composition with shape memory properties comprising bioresorbable polymer of the present invention or of item 4.

Item 8 is a bioresorbable polymer of the present invention or of item 3, wherein the shape changes in a temperature range of-60 to 60°C, preferably in a temperature range of 0 to 59°C, and more preferably in a temperature range of 1 to 45°C.

Item 9 is a vascular closure device comprising a composition of the present invention or of item 3.

Item 9a is a vascular closure device comprising a composition of item 3, item 4, item 6 or item 7.

Item 10 is a stent comprising a composition of the present invention or of item 3.

Item 10a is a stent comprising a composition of item 3, item 4, item 6 or item 7.

Item 11 is a mastectomy device comprising a composition of the present invention or of item 3.

Item 11a is a mastectomy device comprising a composition of item 3, item 4, item 6 or item 7.

Item 12 is a fast degrading bioresorbable polymer composition comprising a bioresorbable polymer of the present invention or from item 3.

Item 12a is a fast degrading bioresorbable polymer composition comprising a bioresorbable polymer from item 3, item 4, item 6 or item 7.

Item 13 is a 3D printed part comprising a composition of the present inventio or of item 3.

Item 13a is a 3D printed part comprising a composition of item 3, item 4, item 6 or item 7.

Item 14 is a process for producing a 3D printed part containing a bioresorbable polymer of item 1 or item 2, or the composition of the present invention, or a bioresorbable polymer of item 3 using a solution based 3D printing machine; the process comprising:
(a) providing bioresorbable polymer of Formula A of item 1, Formula B of item 2, or a mixture thereof or the composition of the present invention;
(b) adding bioresorbable polymer of Formula A of item 1, Formula B of item 2, or a mixture thereof or the composition of the present invention to a solvent to form a polymer solution;
(c) printing the polymer solution through a print head to form the 3D printed part by depositing sequential layers onto the build stage of the printer.

Item 15 is a process for producing a 3D printed part containing a bioresorbable polymer composition of the present inventio or of item 3 using an extrusion based 3D printing machine, wherein the process comprises printing the polymer or mixture through a print head to form the 3D printed part, and depositing sequential layers onto the build stage of the printer.

Item 16 is a process of producing a 3D printed part using binderjetting, comprising the steps of:
(a) providing powder of bioresorbable polymer composition of the present invention or of Item 3;
(b) selectively depositing an amount of a binder onto the powder of polymer of the present invention or of Item 3 to produce an unfinished layer;
(c) repeating steps (a) and (b) to produce a three-dimensional unfinished model; and
(d) sintering the unfinished model to produce a three-dimensional 3D printed part.

Item 17 is the 3D printed part of item 13 having a functional gradient along the build axis of the part.

Item 18 is use of 3D printed parts of item 13 as bone fillers, vascular closure device, and hemostasis device.

Item 18a is use of 3D printed parts of items 13 and 17 as bone fillers, vascular closure device, and hemostasis device.

Item 19 is use of 3D printed parts of item 13 in aneurysms applications, mastectomy application, and stent applications.

Item 19a is use of 3D printed parts of items 13 and 17 in aneurysms applications, mastectomy application, and stent applications.

Item 20 is use of 3D printed parts of item 13 as a scaffold for regenerative medicine.

Item 20a is use of 3D printed parts of items 13 and 17 as a scaffold for regenerative medicine.

## Claims

1. A bioresorbable polymer of Formula A 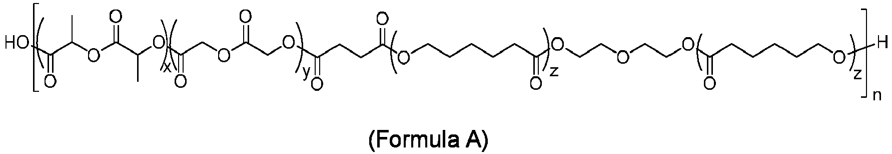 wherein:
x is between 0 and 2,000;
y is between 0 and 2,000;
x plus y is between 2 and 2000;
z is between 2 and 2,000; and
n is between 1 and 1,000.

2. A bioresorbable polymer of Formula B 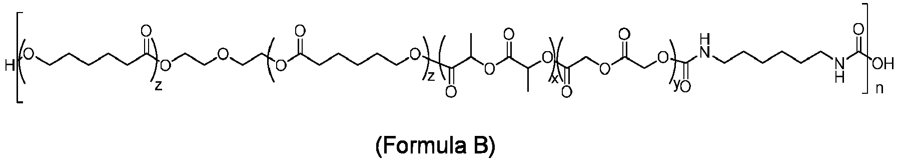 wherein:
x is between 0 and 2,000;
y is between 0 and 2,000;
x plus y is between 2 and 2000;
z is between 2 and 2,000; and
n is between 1 and 1,000.

3. The bioresorbable polymer of claim 1 or 2, wherein the shape of the bioresorbable polymer changes in a temperature range of -60 to 60°C.

4. A composition comprising:
a bioresorbable polymer of Formula A of claim 1 or 3, or of Formula B of claim 2 or 3, or a mixture thereof.

5. The composition of claim 4, wherein the composition further comprises an additive, wherein the additive is an antimicrobial agent, an antibacterial agent, a growth factor, a vitamin, a biologic, an antibiotic, an antiviral agent, antifungal agent, alendronate, olpadronate, etidronate, colecalciferol, tocopherol, pyridoxin, cobalamine platelet-derived growth factor, glycine, lysine, penicillin, cephalosporin, lamivudine, tetracycline, and zidovudine, polyethylene glycol, a polyamino acid, an aliphatic polyester, a saccharide, a polysaccharide, an aliphatic polycarbonate, a poly amine, a polyanhydride, a steroid, glycerol, ascorbic acid, an amino acid, or a peptide, an inorganic particle, poly-I-lysine, poly-d-lysine, poly-d,I-lysine, poly-I-cysteine, poly-d-cysteine, poly-d,I-cysteine, short oligomers of I-lysine, d-lysine, I- cysteine, d-cysteine, an amino functionalized PEG, an amino functionalized inorganic particle, a tin catalyst, or a mixture thereof.

6. A process for preparing a bioresorbable polymer of Formula A of claim 1 or 3 or a bioresorbable polymer of Formula B of claim 2 or 3 comprising the steps of mixing a polymer backbone with a functional group precursor to form a mixture; and adding a linker to the mixture to form the bioresorbable polymer.

7. A vascular closure device comprising a composition of claim 4 or 5.

8. A stent comprising a composition of claim 4 or 5.

9. A mastectomy device comprising a composition of claim 4 or 5.

10. A 3D printed part comprising a composition of claims 4 or 5.

11. A process for producing a 3D printed part containing a polymer of any of claims 1 to 3 or the composition of claim 4 or 5 using a solution based 3D printing machine; the process comprising:
(a) providing the bioresorbable polymer of Formula A of claim 1 or 3, Formula B of claim 2 or 3, or a mixture thereof or the composition of claim 4 or 5;
(b) adding the bioresorbable polymer of Formula A, Formula B , or a mixture thereof or the composition to a solvent to form a polymer solution;
(c) printing the polymer solution through a print head to form the 3D printed part by depositing sequential layers onto the build stage of the printer.

12. A process for producing a 3D printed part containing a bioresorbable polymer of any of claims 1 to 3 or the composition of claim 4 or 5 using an extrusion based 3D printing machine, wherein the process comprises printing the polymer or mixture thereof or the composition through a print head to form the 3D printed part, and depositing sequential layers onto the build stage of the printer.

13. A process of producing a 3D printed part using binderjetting, comprising the steps of:
(a) providing powder of a bioresorbable polymer of any of claims 1 to 3 or the composition of claim 4 or 5;
(b) selectively depositing an amount of a binder onto the powder of polymer or the composition to produce an unfinished layer;
(c) repeating steps (a) and (b) to produce a three-dimensional unfinished model; and
(d) sintering the unfinished model to produce a three-dimensional 3D printed part.

14. The 3D printed part of claim 10 having a functional gradient along the build axis of the part.

15. Use of 3D printed parts of claim 10 as bone fillers, vascular closure device, and hemostasis device, in aneurysms applications, mastectomy application, and stent applications, or as a scaffold for regenerative medicine.

## Patentansprüche

1. Bioresorbierbares Polymer der Formel A 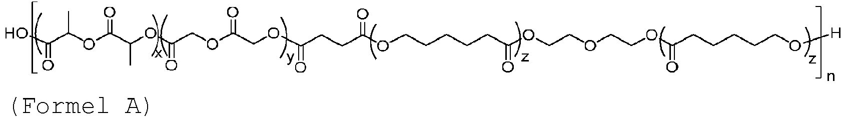 wobei:
x zwischen 0 und 2.000 beträgt;
y zwischen 0 und 2.000 beträgt;
x plus y zwischen 2 und 2000 beträgt;
z zwischen 2 und 2.000 beträgt; und
n zwischen 1 und 1.000 beträgt.

2. Bioresorbierbares Polymer der Formel B 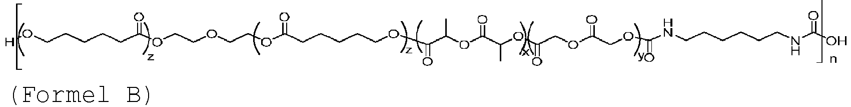 wobei:
x zwischen 0 und 2.000 beträgt;
y zwischen 0 und 2.000 beträgt;
x plus y zwischen 2 und 2000 beträgt;
z zwischen 2 und 2.000 beträgt; und
n zwischen 1 und 1.000 beträgt.

3. Bioresorbierbares Polymer gemäß Anspruch 1 oder 2, wobei sich die Form des bioresorbierbaren Polymers in einem Temperaturbereich von -60 bis 60 °C verändert.

4. Zusammensetzung umfassend:
ein bioresorbierbares Polymer der Formel A gemäß Anspruch 1 oder 3 oder der Formel B gemäß Anspruch 2 oder 3 oder ein Gemisch davon.

5. Zusammensetzung gemäß Anspruch 4, wobei die Zusammensetzung ferner einen Zusatzstoff umfasst, wobei der Zusatzstoff ein antimikrobielles Mittel, ein antibakterielles Mittel, ein Wachstumsfaktor, ein Vitamin, ein biologischer Stoff, ein Antibiotikum, ein antivirales Mittel, antifungielles Mittel, Alendronat, Olpadronat, Etidronat, Colecalciferol, Tocopherol, Pyridoxin, Cobalamin plättchenabgeleiteter Wachstumsfaktor, Glycin, Lysin, Penicillin, Cephalosporin, Lamivudin, Tetracyclin, und Zidovudin, Polyethylenglycol, eine Polyaminosäure, ein aliphatischer Polyester, ein Saccharid, ein Polysaccharid, ein aliphatisches Polycarbonat, ein Polyamin, ein Polyanhydrid, ein Steroid, Glycerol, Ascorbinsäure, eine Aminosäure oder ein Peptid, ein anorganisches Partikel, Poly-l-lysin, Poly-d-lysin, Poly-d,l-lysin, Poly-l-cystein, Poly-d-cystein, Poly-d, l-cystein, kurze Oligomere von l-Lysin, d-Lysin, l-Cystein, d-Cystein, ein aminofunktionalisiertes PEG, ein aminofunktionalisiertes anorganisches Partikel, ein Zinnkatalysator oder ein Gemisch davon ist.

6. Verfahren zur Herstellung eines bioresorbierbaren Polymers der Formel A gemäß Anspruch 1 oder 3 oder eines bioresorbierbaren Polymers der Formel B gemäß Anspruch 2 oder 3, umfassend die Schritte des Mischens eines Polymergerüsts mit einem Vorläufer einer funktionellen Gruppe, um ein Gemisch zu bilden; und Zugeben eines Linkers zu dem Gemisch, um das bioresorbierbare Polymer zu bilden.

7. Gefäßverschlussvorrichtung, umfassend eine Zusammensetzung gemäß Anspruch 4 oder 5.

8. Stent, umfassend eine Zusammensetzung gemäß Anspruch 4 oder 5.

9. Mastektomievorrichtung, umfassend eine Zusammensetzung gemäß Anspruch 4 oder 5.

10. 3D-Gedrucktes Teil, umfassend eine Zusammensetzung gemäß Anspruch 4 oder 5.

11. Verfahren zur Herstellung eines 3D-gedruckten Teils, das ein Polymer gemäß einem der Ansprüche 1 bis 3 oder die Zusammensetzung gemäß Anspruch 4 oder 5 enthält, unter Verwendung einer 3D-Druckmaschine auf Lösungsbasis; wobei das Verfahren umfasst:
(a) Bereitstellen des bioresorbierbaren Polymers der Formel A gemäß Anspruch 1 oder 3, Formel B gemäß Anspruch 2 oder 3, oder eines Gemischs davon, oder der Zusammensetzung gemäß Anspruch 4 oder 5;
(b) Zugeben des bioresorbierbaren Polymers der Formel A, Formel B oder eines Gemischs davon oder der Zusammensetzung zu einem Lösungsmittel, um eine Polymerlösung zu bilden;
(c) Drucken der Polymerlösung durch einen Druckkopf, um das 3D-gedruckte Teil durch Abscheiden aufeinanderfolgender Schichten auf die Bauplattform des Druckers zu bilden.

12. Verfahren zur Herstellung eines 3D-gedruckten Teils, das ein bioresorbierbares Polymer gemäß einem der Ansprüche 1 bis 3 oder die Zusammensetzung gemäß Anspruch 4 oder 5 enthält, unter Verwendung einer 3D-Druckmaschine auf Extrusionsbasis, wobei das Verfahren Drucken des Polymers oder Gemischs davon oder der Zusammensetzung durch einen Druckkopf, um das 3D-gedruckte Teil zu bilden, und Abscheiden aufeinanderfolgender Schichten auf die Bauplattform des Druckers umfasst.

13. Verfahren zur Herstellung eines 3D-gedruckten Teils unter Verwendung von Binder-Jetting, umfassend die Schritte:
(a) Bereitstellen von Pulver eines bioresorbierbaren Polymers gemäß einem der Ansprüche 1 bis 3 oder der Zusammensetzung gemäß Anspruch 4 oder 5;
(b) selektives Abscheiden einer Menge eines Bindemittels auf das Pulver des Polymers oder der Zusammensetzung, um eine unfertige Schicht zu erzeugen;
(c) Wiederholen der Schritte (a) und (b), um ein dreidimensionales unfertiges Modell zu erzeugen; und
(d) Sintern des unfertigen Modells, um ein dreidimensionales 3D-gedrucktes Teil zu erzeugen.

14. 3D-gedrucktes Teil gemäß Anspruch 10 mit einem funktionellen Gradienten entlang der Bauachse des Teils.

15. Verwendung von 3D-gedruckten Teilen gemäß Anspruch 10 als Knochenfüller, Gefäßverschlussvorrichtung und Hämostasevorrichtung, bei Aneurysmaanwendungen, Mastektomieanwendungen und Stentanwendungen oder als Gerüst für regenerative Medizin.

## Revendications

1. Polymère biorésorbable de formule A 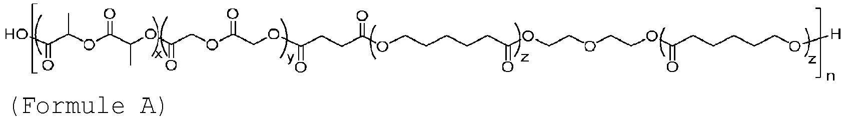 dans laquelle
x est compris entre 0 et 2 000 ;
y est compris entre 0 et 2 000 ;
x plus y est compris entre 2 et 2 000 ;
z est compris entre 2 et 2 000 ; et
n est compris entre 1 et 1 000.

2. Polymère biorésorbable de formule B 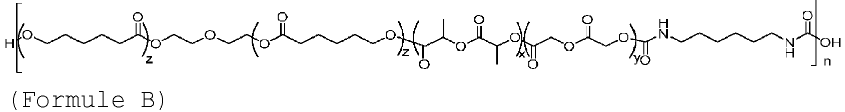 dans laquelle
x est compris entre 0 et 2 000 ;
y est compris entre 0 et 2 000 ;
x plus y est compris entre 2 et 2 000 ;
z est compris entre 2 et 2 000 ; et
n est compris entre 1 et 1 000.

3. Polymère biorésorbable selon la revendication 1 ou 2, dans lequel la forme du polymère biorésorbable change dans une plage de températures de -60 à 60 °C.

4. Composition comprenant :
un polymère biorésorbable de formule A selon la revendication 1 ou 3, ou de formule B selon la revendication 2 ou 3, ou un mélange de celles-ci.

5. Composition selon la revendication 4, la composition comprenant en outre un additif, l'additif étant un agent antimicrobien, un agent antibactérien, un facteur de croissance, une vitamine, un produit biologique, un antibiotique, un agent antiviral, un agent antifongique, l'alendronate, l'olpadronate, l'étidronate, le colécalciférol, le tocophérol, la pyridoxine, la cobalamine, le facteur de croissance dérivé des plaquettes, la glycine, la lysine, la pénicilline, la céphalosporine, la lamivudine, la tétracycline et la zidovudine, le polyéthylèneglycol, un polyaminoacide, un polyester aliphatique, un saccharide, un polysaccharide, un polycarbonate aliphatique , une polyamine, un polyanhydride, un stéroïde, le glycérol, l'acide ascorbique, un acide aminé ou un peptide, une particule inorganique, la poly-l-lysine, la poly-d-lysine, la poly-d,l-lysine, la poly-l-cystéine, la poly-d-cystéine, la poly-d,l-cystéine, des oligomères courts de l-lysine, de d-lysine, de l-cystéine, de d-cystéine, un PEG à fonctionnalité amino, une particule inorganique à fonctionnalité amino, un catalyseur à l'étain, ou un mélange de ceux-ci.

6. Procédé de préparation d'un polymère biorésorbable de formule A selon la revendication 1 ou 3 ou d'un polymère biorésorbable de formule B selon la revendication 2 ou 3, comprenant les étapes de mélange d'un squelette polymère avec un précurseur de groupe fonctionnel pour former un mélange ; et ajout d'un lieur au mélange pour former le polymère biorésorbable.

7. Dispositif de fermeture vasculaire comprenant une composition selon la revendication 4 ou 5.

8. Stent comprenant une composition selon la revendication 4 ou 5.

9. Dispositif de mastectomie comprenant une composition selon la revendication 4 ou 5.

10. Pièce imprimée en 3D comprenant une composition selon les revendications 4 ou 5.

11. Procédé de production d'une pièce imprimée en 3D contenant un polymère selon l'une quelconque des revendications 1 à 3 ou la composition selon la revendication 4 ou 5 au moyen d'une machine d'impression 3D à base de solution ; le procédé comprenant :
(a) la fourniture du polymère biorésorbable de formule A selon la revendication 1 ou 3, de formule B selon la revendication 2 ou 3, ou d'un mélange de celles-ci ou de la composition selon la revendication 4 ou 5 ;
(b) l'ajout du polymère biorésorbable de formule A, de formule B, ou d'un mélange de celles-ci ou de la composition à un solvant pour former une solution de polymère ;
(c) l'impression de la solution de polymère par l'intermédiaire d'une tête d'impression pour former la pièce imprimée en 3D par dépôt de couches successives sur la plaque de construction de l'imprimante.

12. Procédé de production d'une pièce imprimée en 3D contenant un polymère biorésorbable selon l'une quelconque des revendications 1 à 3 ou la composition selon la revendication 4 ou 5 au moyen d'une machine d'impression 3D à base d'extrusion, le procédé comprenant l'impression du polymère ou d'un mélange correspondant ou de la composition par l'intermédiaire d'une tête d'impression pour former la pièce imprimée en 3D et le dépôt de couches séquentielles sur la plaque de construction de l'imprimante.

13. Procédé de production d'une pièce imprimée en 3D au moyen d'une projection de liant, comprenant les étapes de :
(a) la fourniture d'une poudre d'un polymère biorésorbable selon l'une quelconque des revendications 1 à 3 ou de la composition selon la revendication 4 ou 5 ;
(b) le dépôt sélectif d'une quantité d'un liant sur la poudre de polymère ou la composition pour produire une couche brute ;
(c) la répétition des étapes (a) et (b) pour produire un modèle tridimensionnel brut ; et
(d) le frittage du modèle brut pour produire une pièce imprimée en 3D tridimensionnelle.

14. Pièce imprimée en 3D selon la revendication 10 présentant un gradient fonctionnel le long de l'axe de construction de la pièce.

15. Utilisation de pièces imprimées en 3D selon la revendication 10 en tant que produits de comblement osseux, dispositif de fermeture vasculaire et dispositif d'hémostase, dans des applications d'anévrisme, des application de mastectomie et des applications de stent, ou en tant qu'échafaudage pour la médecine régénérative.
